# EUROPEAN PATENT SPECIFICATION

(11) **EP 1 866 432 B1**
(45) Date of publication and mention of the grant of the patent: **05.08.2009**
(21) Application number: 06734173.5
(22) Date of filing: 31.01.2006
(51) Int. Cl.: C12Q 1/26, C12M 1/34

(54) **RAPID-RESPONSE GAS SENSING ELEMENT**
SCHNELL REAGIERENDES GASWAHRNEHMUNGSELEMENT
ÉLÉMENT DE DÉTECTION DE GAZ À RÉPONSE RAPIDE

(30) Priority: 05.02.2005 US 53253
(43) Date of publication of application: 19.12.2007
(73) Proprietor: Apieron Inc., Menlo Park, California 94025 (US)
(72) Inventor: ANVAR, David, J., Sunnyvale, California 94087 (US); PARIKH, Bhairavi, R., Palo Alto, California 94306 (US); FAY, Jonathan, San Mateo, California 94402 (US); CHAZAN, David, J., Palo Alto, California 94306-3134 (US)
(74) Representative: Jacob, Reuben Ellis
(86) International application number: PCT/US2006/003579
(87) International publication number: WO 2006/086197

(56) References cited:
- US-A- 5 200 334
- US-B2- 6 824 776
- AYLOTT J W ET AL: "Optical Biosensing of Gaseous Nitric Oxide Using Spin-Coated Sol-Gel Thin Films" CHEMISTRY OF MATERIALS, AMERICAN CHEMICAL SOCIETY, WASHINGTON, US, vol. 9, no. 11, 1997, pages 2261-2263, XP003009532 ISSN: 0897-4756
- WALLACE J M ET AL: "Silica nanoarchitectures incorporating self organising protein superstructures with gas phase bioactivity" NANO LETTERS, vol. 3, no. 10, 16 September 2003 (2003-09-16), pages 1463-1467, XP002470205

## Description

This invention relates to sensing elements for measuring the concentration of gaseous substances.

Analysis of a subject's exhaled breath is a valuable clinical tool, with applications in the diagnosis and management of many conditions. A change in nitric oxide (NO) concentration in exhaled breath, for example, can indicate a change in the level of inflammation in the airway of an asthmatic, which in tum can indicate an increase in the likelihood of an asthma attack. Excessive carbon monoxide (CO) can indicate hemolytic jaundice, and high levels of hydrogen can indicate carbohydrate malabsorption.

Various sensors have been developed to measure the concentrations of different gaseous analytes. Some of these sensors detect and measure changes in bioactive substances, which can hence be termed "chemical transducers," in response to the analyte. The chemical transducer, or sensing element, is the chemical component of the sensor that undergoes a detectable change in state or other property, the detectable change being one that can be measured directly. One such sensor measures optically quantifiable changes in a sol-gel encapsulated cytochrome c in response to NO. This sensor and related technology are disclosed in the following U.S. published patent applications and patents, US 2004-0017570 A1, published January 29, 2004 (Application No. 10/334,625, filed December 30, 2002); US 2005-0053549 A1, published March 10, 2005 (Application No. 10/659,408, filed September 10,2003); and US 2005-0083527 A1, published April 21, 2005 (Application No. 10/767,709, filed January 28, 2004) ; U.S. Patent No. 5,795.187, issued August 18, 1998; U.S. Patent No. 6,010,459, issued January 4, 2000; and US-B2-6824776.

Any commercially viable sensor for detecting trace levels of a gaseous analyte must have a sensing element that responds rapidly to the analyte. Rapid response to trace gases requires a sensor that readily allows the gas to reach the sensing element, that generates a large signal in proportion to the number of gas molecules contacting the element, and that demonstrates high specificity to the analyte to be detected. The sensing element may, for example, contain an optically active protein that specifically binds the analyte and that, as a result of the binding, undergoes a change in the optical absorbance spectrum of the protein that can be directly detected and measured. Proteins are particularly useful sensing elements due to their extraordinary specificity and efficient transduction (i.e., signal generation, such as optical absorbance). Where applicable, therefore, proteins are often a preferred transducer for the analysis of trace gases.

Considerations in developing a protein-based gas sensor include a) the immobilization or encapsulation of the protein while retaining its activity (i.e., avoiding denaturation of the protein), b) the choice of a support matrix (i.e., a host material) that preserves the activity of the protein during storage and that has a sufficient porosity to provide ready access of the sample gas to the protein, c) the determination of an optimum protein concentration, i.e., one that is high enough to generate a strong response to the analyte but not so high that the protein impedes travel of the sample gas through the matrix, and d) the determination of the state of the protein in which the protein will perform is detection function most effectively.

The protein should be immobilized in a host material (support matrix) without loss of activity due to the unfolding or rupture of peptide bonds. Each protein has a different susceptibility to denaturation and will have a sensitivity that varies due to a number of factors. These include the pH that is maintained during the preparation of the support matrix, particularly when the matrix is a sol-gel, the choice and concentration of solvents, the concentration of ions and additives, and finally the temperature imposed during the immobilization of the protein in the host material.

The host material must (i) be compatible with the protein, (ii) have the appropriate chemical functionality on its surface, (iii) have a sufficiently high porosity to permit rapid analyte diffusion in and out of the sensor, and (iv) be suitable to the interrogation method used for detection. To be compatible, the host material must not have any chemical functionality that can react adversely with the immobilized protein, or decompose into products that will react adversely with the protein. In addition to chemical activity, the chemical functionality (hydrophilicity or hydrophobicity, for example) of the surface must not induce changes in the protein conformation that are large enough to result in the loss of the intrinsic functionality of the protein. Finally, the host material should be porous enough to allow the gaseous analyte to rapidly diffuse to the protein, and to allow the analyte, or an undesired reactive byproduct of the analyte that is reactive with the protein, to diffuse out of the host material. In addition, the host material pore size must be large enough for the protein to remain inside without being deformed either initially or during the post processing of the host material.

The present invention resides in a gas sensing element and a method of manufacturing the sensing element. In one embodiment, the sensing element includes cytochrome c embedded in a sol-gel matrix. The sol-gel matrix may take the form of a thin film or a monolith. The invention also resides in the use and adjustment of various parameters for creating a sensing element that provides effective results for trace gas analysis. These parameters include protein concentration, sol-gel pore size, and, for monolith-fomi elements, surface area of the monolith.

In one embodiment of the invention, cytochrome c is incorporated into a sol-gel glass with methanol at a concentration of from about 15% by volume about 40% by volume, with an ammonia catalyst at a concentration of from about 0.02 N to about 0.2 N, and held at a temperature of about 55°C or below. A pre-polymer is included in the solution, and the protein is immobilized by entrapment within a polymer network that forms around the protein as the pre-polyner is polymerized.

The resulting sol-gel is porous and hydrophilic, retaining enough water and having a sufficiently constrained pore geometry to promote the stability of the cytochrome c, even at temperatures exceeding the denaturation temperature of the protein. The denaturation temperature of the protein in solution is reported in the literature, and when encapsulated in the support matrix in accordance with this invention, the protein will remain functional at 15 to 20 degrees Celsius above this temperature. The pore width (also referred to as the pore diameter) of the sol-gel preferably ranges from 3.0 to 6.0 nm, and most preferably from 3.5 to 5.8 nm, and the surface area preferably ranges from about 550 m²/g to about 650 m²/g.

The optimum protein concentration is determined by the expected concentration of the trace gas to be detected, the aspect ratio of the sensor matrix, the mean pore width of the matrix, and the detection method. Examples of detection methods are optical absorbance spectroscopy, fluorescence, and chemiluminescence, as well as coulometric, potentiometric, impedance, and piezoelectric methods.

For trace gas analysis, the maximum protein concentration that can be retained by the matrix is rarely the optimum protein concentration for purposes of rapid and accurate analysis, especially when the expected analyte concentration is close to, or of the same order of magnitude as, the protein concentration. The optimum protein concentration may also vary with the host matrix aspect ratio because diffusion in a porous medium can be dependent on path length if long-range connectivity is limited beyond a percolation threshold.
Excessive protein concentration can limit the transport of the analyte, especially when the size of the protein is of the same order of magnitude as the pore width.

The optimum protein concentration is a function of pore width, the tortuosity of the medium, the size of the protein, and the colligative properties of the protein during the formation of the polymer network. A high protein concentration can also affect the background level and the signal-to-noise ratio. When detection is performed by optical absorbance spectroscopy, for example, a protein concentration that is too low will result in very high background light level and a small signal, while a protein concentration that is too high will result in a poor signal-to-noise ratio and other potential problems associated with the need to increase the light source output, such as temperature control, photo-bleaching, non-linear responses to analyte concentration, and others. As explained below, a preferred final xerogel concentration for a cytochrome c/sol-gel sensing element in certain embodiments of the invention is in the approximate range of 20 mg/cm³ to 27 mg/cm³. In other embodiments, the preferred final xerogel concentration is within the approximate range of 8 mg/cc to 15 mg/cm³, most preferably within the approximate range of 10 mg/cm³ to 12 mg/cm³, with a value of approximately 10 mg/cm³ particularly preferred.

The sensing element can be in the form of a thin film, and in one embodiment this film is about 600 nm in thickness with a cytochrome c concentration of about 1 mM (about 12 mg/cm³) or less. Upon shrinkage of the sol-gel by a factor of approximately 2.2 in a linear dimension, and with shrinkage of a thin film occurring only in a direction normal to the surface of the sol-gel, the protein concentration rises to about 2.2 mM or 26.4 mg/cm³ of cytochrome C in the resulting xerogel film.

In another embodiment, the sol-gel is a monolith, preferably with a peak optical density of about 2.8 absorbance units at 400 nm. In the practice of this invention, the monolith detects nitric oxide in exhaled breath at NO levels of 200 ppb or less, preferably 100 ppb or less, and most preferably 50 ppb or less. Within its limits of detection, the monolith can indicate an absence of NO, or in some cases a lower limit of 5 ppb or 10 ppb. A monolith that is prepared from a solution containing 0.092 mM or 1.1 mg/cm³ of cytochrome c will respond quickly, i.e., in less than one minute, to nitric oxide at concentrations below 50 ppb. When the solution concentration of cytochrome c is raised to 0.46 mM or 5.7 mg/cm³, the response rate decreases to about one-tenth of their value at the lower concentration. The preferred monolith has a pore network with a pore width of from 3.0 nm to 6.0 nm, preferably from 3.5 nm to 5.8 nm, and a surface area of about 550 to about 650 m²/g. Upon shrinkage of the monolith from a 0.092 mM cytochrome c solution during curing and drying, the cytochrome c concentration rises to (2.2)³ × 0.092 = 0.98 mM or (2.2)³ × 1.1 = 11.71 1 mg/cm³. These concentrations approach, but do not equal, the maximum concentration of cytochrome c allowable for a fast-responding monolith. With this in mind, the maximum allowable concentrations of cytochrome c in fast-responding thin films and monoliths for trace gas sensors are both close to 2 mM or approximately 25 mg/cm³ in the final xerogel state. The term "trace gas" is used herein to denote a gas at a concentration in the ppb range, preferably, as noted above, 50 ppb or less.

When the protein concentration is reduced, the degree of folding in the protein is reduced. In this condition, known in the art as the molten globule state, one of the axial ligands of the heme core of the protein are further removed on average from the iron center, an effect that is believed to result in an opening of the protein to provide greater access to nitric oxide binding. A discovery in accordance with the present invention is that the changes in the process parameters that increase the degree of protein folding and the amount of retained water also decrease the responsiveness of the sensor. Increasing the protein concentration by factors of 5 and 10, for example, are observed to place the protein in a more folded state and to reduce the rate of response to nitric oxide.

In summary, relatively low protein concentrations are beneficial in the practice of this invention, since the greater colligative properties of the protein at higher concentrations cause increased clogging of the pore network and less protein-glass interaction. The clogged network reduces the mobility of the analyte gas through the thin film or monolith, and the increased protein crowding and hydration reduce the rate of binding of the nitric oxide to the cytochrome c.

One skilled in the art will appreciate that the present invention can be practiced by other than the preferred embodiments, which are presented herein for purposes of illustration and not of limitation.

## Claims

1. A method for detecting nitric oxide in a sample of exhaled breath at a concentration of 200 ppb or less, said method comprising:
contacting said exhaled breath in the gas phase with a xerogel monolith comprising cytochrome c encapsulated in a polymeric matrix, said xerogel monolith having a cytochrome c concentration of 1 mg/cm³ to 40 mg/cm³, a pore diameter of 3.0 nm to 6.0 nm, and a surface area of 550 m²/g to 650 m²/g;
detecting a change in an optical property of said monolith as an indication of the binding of said nitric oxide to said cytochrome c; and
correlating any change so detected with the concentration of nitric oxide in said exhaled breath.

2. The method of claim **1** wherein said nitric oxide concentration in said exhaled breath is 100 ppb or less.

3. The method of claim **1** wherein said nitric oxide concentration in said exhaled breath is 50 ppb or less.

4. The method of claim **1** wherein said pore diameter is 3.5 nm to 5.8 nm.

5. The method of claim **1** wherein said cytochrome c concentration is from 20 mg/cm³ to 27 mg/cm³.

6. The method of claim **1** wherein said cytochrome c concentration is 25 mg/cm³ or less.

7. The method of claim **1** wherein said cytochrome c concentration is from 8 mg/cm³ to 15 mg/cm³.

8. The method of claim **1** wherein said cytochrome c concentration is from 10 mg/cm³ to 12 mg/cm³.

9. The method of claim **1** wherein said monolith has a peak optical density of about 2.8 absorbance units at 400 nm.

10. The method of claim **1** wherein said optical property is optical absorbance.

## Patentansprüche

1. Verfahren zum Erfassen von Stickoxid in einer Probe ausgeatmeter Luft mit einer Konzentration von 200 ppb oder weniger, wobei das Verfahren umfasst:
Inkontaktbringen der ausgeatmeten Luft in der Gasphase mit einem Xerogelmonolith, umfassend Cytochrom c, das in einer Polymermatrix eingekapselt ist, wobei der Xerogelmonolith eine Cytochrom c-Konzentration von 1 mg/cm³ bis 40 mg/cm³, einen Porendurchmesser von 3,0 nm bis 6,0 nm und eine Oberfläche von 550 m²/g bis 650 m²/g aufweist;
Erfassen einer Änderung in einer optischen Eigenschaft des Monolithen als Hinweis auf die Bindung von Stickoxid mit dem Cytochrom c; und
Korrelieren jeder so erfassten Änderung mit der Konzentration von Stickoxid in der ausgeatmeten Luft.

2. Verfahren nach Anspruch 1, wobei die Stickoxidkonzentration in der ausgeatmeten Luft 100 ppb oder weniger beträgt.

3. Verfahren nach Anspruch 1, wobei die Stickoxidkonzentration in der ausgeatmeten Luft 50 ppb oder weniger beträgt.

4. Verfahren nach Anspruch 1, wobei der Porendurchmesser 3,5 nm bis 5,8 nm beträgt.

5. Verfahren nach Anspruch 1, wobei die Cytochrom c-Konzentration zwischen 20 mg/cm³ und 27 mg/cm³ beträgt.

6. Verfahren nach Anspruch 1, wobei die Cytochrom c-Konzentration 25 mg/cm³ oder weniger beträgt.

7. Verfahren nach Anspruch 1, wobei die Cytochrom c-Konzentration zwischen 8 mg/cm³ und 15 mg/cm³ beträgt.

8. Verfahren nach Anspruch 1, wobei die Cytochrom c-Konzentration zwischen 10 mg/cm³ und 12 mg/cm³ beträgt.

9. Verfahren nach Anspruch 1, wobei der Monolith eine optische Spitzendichte von ungefähr 2,8 Absorptionseinheiten mit 400 nm aufweist.

10. Verfahren nach Anspruch 1, wobei die optische Eigenschaft eine optische Absorption ist.

## Revendications

1. Procédé de détection du monoxyde d'azote (ou oxyde nitrique) dans un échantillon d'air exhalé à une concentration de 200 ppb (parties par milliard) ou moins, ledit procédé comprenant :
la mise en contact dudit air exhalé en phase gazeuse avec un monolithe de xérogel comprenant du cytochrome c encapsulé dans une matrice polymère, ledit monolithe de xérogel ayant une concentration en cytochrome c comprise entre 1 mg/cm³ et 40 mg/cm³, un diamètre de pore compris entre 3,0 nm et 6,0 nm, et une aire de surface comprise entre 550 m²/g et 650 m²/g ;
la détection d'un changement d'une propriété optique dudit monolithe comme indication de la liaison dudit monoxyde d'azote audit cytochrome c ; et
la corrélation de tout changement ainsi détecté avec la concentration en monoxyde d'azote dans ledit air exhalé.

2. Procédé selon la revendication 1, dans lequel ladite concentration en monoxyde d'azote dans ledit air exhalé est de 100 ppb ou moins.

3. Procédé selon la revendication 1, dans lequel ladite concentration en monoxyde d'azote dans ledit air exhalé est de 50 ppb ou moins.

4. Procédé selon la revendication 1, dans lequel ledit diamètre de pore est compris entre 3,5 nm à 5,8 nm.

5. Procédé selon la revendication 1, dans lequel ladite concentration en cytochrome c est comprise entre 20 mg/cm³ et 27 mg/cm³.

6. Procédé selon la revendication 1, dans lequel ladite concentration en cytochrome c est de 25 mg/cm³ ou moins.

7. Procédé selon la revendication 1, dans lequel ladite concentration en cytochrome c est comprise entre 8 mg/cm³ et 15 mg/cm³.

8. Procédé selon la revendication 1, dans lequel ladite concentration en cytochrome c est comprise entre 10 mg/cm³ et 12 mg/cm³.

9. Procédé selon la revendication 1, dans lequel ledit monolithe possède une densité optique maximale d'environ 2,8 unités d'absorbance à 400 nm.

10. Procédé selon la revendication 1, dans lequel ladite propriété optique est l'absorbance optique.
